# EUROPÄISCHE PATENTANMELDUNG

(11) **EP 1 133 981 A2**
(43) Veröffentlichungstag der Anmeldung: **19.09.2001**
(21) Anmeldenummer: 01106118.1
(22) Anmeldetag: 13.03.2001
(51) Int. Cl.: A61K 7/42

(54) **Verwendung von Lichtschutzmittelkombinationen, die als wesentlichen Bestandteil 4,4-Diarylbutadiene enthalten als photostabile UV-Filter in kosmetischen und pharmazeutischen Zubereitungen**

(30) Priorität: 15.03.2000 DE 10012413
(71) Anmelder: BASF AKTIENGESELLSCHAFT, 67056 Ludwigshafen (DE)
(72) Erfinder: Heidenfelder, Thomas, Dr., 67354 Römerberg (DE); Habeck, Thorsten, Dr., 67149 Meckenheim (DE); Wünsch, Thomas, Dr., 67346 Speyer (DE)

(57) **Zusammenfassung**

Verwendung von Lichtschutzmittelkombinationen, enthaltend
A) im wesentlichen im UV-A-Bereich absorbierende und
B) weitere im UV-A-Bereich, im UV-B-Bereich und über beide Bereiche absorbierende Verbindungen,
   wobei die im UV-A-Bereich absorbierenden Bestandteile wirksamen Mengen von mindestens

A) einem 4,4'-Diarylbutadien der Formel I 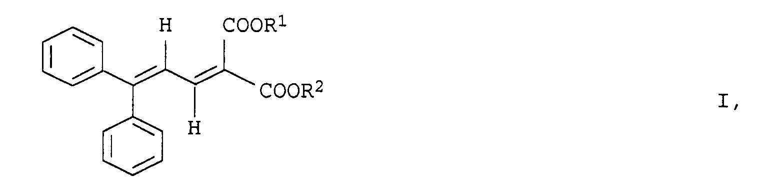 in der
   R¹ und R² unabhängig voneinander Wasserstoff, C₁-C₂₀-Alkyl, C₃-C₁₀-Cycloalkyl oder C₃-C₁₀-Cycloalkenyl bedeuten,
   aufweisen und
   als Bestandteile
B) wirksame Mengen mindestens einer Verbindung enthalten, ausgewählt aus der Gruppe bestehend aus
   Ba) Dibenzoylmethanverbindungen der Formel III 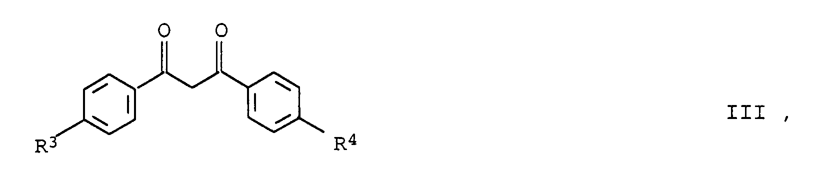 in der
      R³ C₁-C₁₂-Alkyl und
      R⁴ Wasserstoff, C₁-C₁₂-Alkyl oder C₁-C₁₂-Alkoxy bedeuten,
   Bb) Triazinderivaten der Formel IV 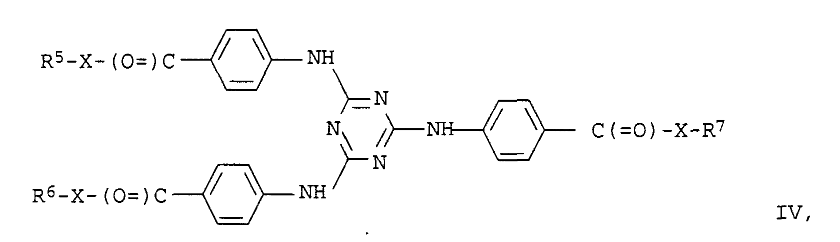
      R⁵ bis R⁷ unabhängig voneinander gegebenenfalls substituiertes C₁-C₂₀-Alkyl, C₅-C₁₀-Aryl, C₅-C₁₀-Heteroaryl oder SpSil bedeuten, wobei Sp für einen Spacer und Sil für einen Silan-, Oligosiloxan- oder Polysiloxanrest steht,
      X für die zweiwertigen Reste
         ―O― oder 〉N―R⁸ steht, wobei
      R⁸ Wasserstoff oder gegebenenfalls substituiertes C₁-C₂₀-Alkyl, C₅-C₁₀-Aryl oder C₅-C₁₀-Heteroaryl bedeutet,
   Bc) Triazinderivate der Formel V 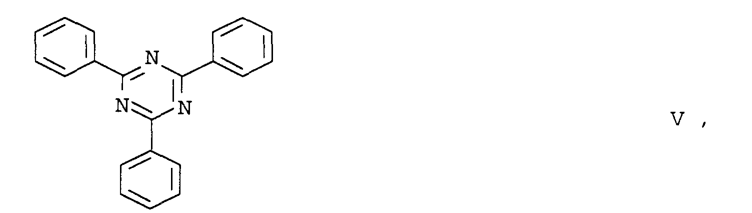 in der an die Phenylringe mindestens eine o-Hydroxygruppe und mindestens eine p-Alkoxygruppe mit 1 bis 20 C-Atomen gebunden sind,
   Bd) dem Benztriazolderivat der Formel VI 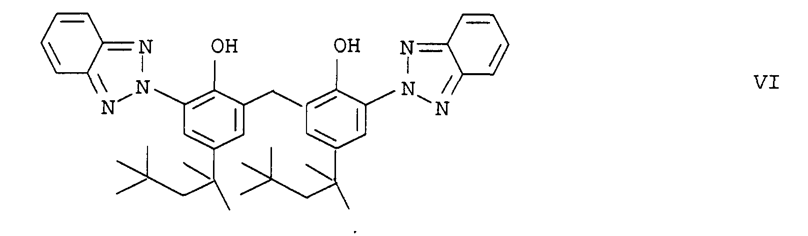
   Be) dem Benzimidazolderivat der Formel VII 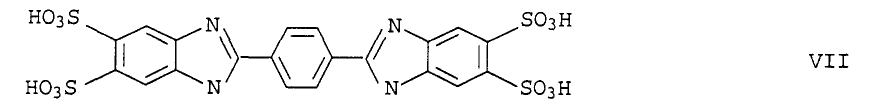 und Salze
   Bf) dem Benztriazolderivat der Formel VIII 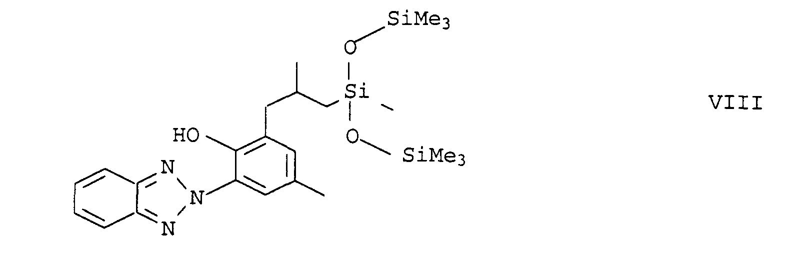
   Bg) o,o',p,p'-Tetrahydroxybenzophenon der Formel IX 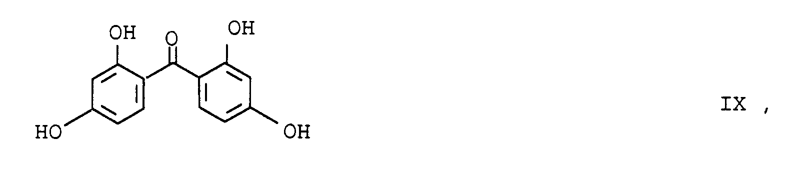
   Bh) einem Organosiloxanbenzalmalonat der Formel Xa 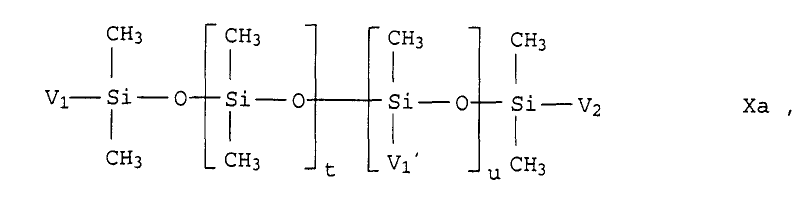 in der
      V₁' die Gruppe 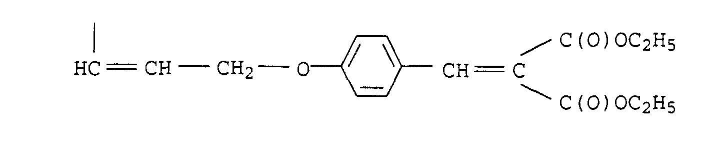 V₁ eine Methylgruppe oder V₁' bedeutet oder
      der Formel Xb 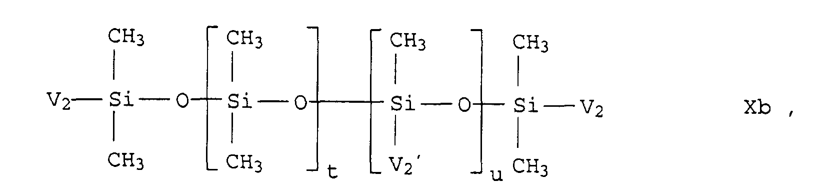 in der V₂' die Gruppe der Struktur 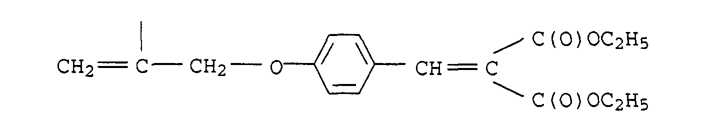 V₂ eine Methylgruppe oder V₂' bedeutet
      oder Mischungen aus Verbindungen der Formeln Xa und Xb,
wobei t für einen Wert bis 100 und u für einen Wert bis 20 steht mit der Maßgabe, daß u = 0 ist, wenn V₁ = V₁' und/oder V₂ = V'₂ ist und u einen Wert von 1 bis 20 bedeutet, wenn V₁ = CH₃ und/oder V₂ = CH₃ ist,
als photostabile UV-Filter in kosmetischen und pharmazeutischen Zubereitungen zum Schutz der menschlichen Haut der menschlicher Haare gegen Sonnenstrahlen, gegebenenfalls zusammen mit weiteren an sich für kosmetische und pharmazeutische Zubereitungen bekannten, im UV-Bereich absorbierenden Verbindungen.

## Beschreibung

Die Erfindung betrifft die Verwendung von Lichtschutzmittelkombinationen, die als im UV-A-Bereich absorbierenden Bestandteil 4,4'-Diarylbutadiene enthalten und mindestens ein weiteres Lichtschutzmittel, das im UV-A-, im UV-B-Bereich oder in beiden Bereichen absorbiert, ausgewählt aus einer im einzelnen im folgenden definierten Gruppe als photostabile UV-Filter-Kombination in kosmetischen und pharmazeutischen Zubereitungen zum Schutz der menschlichen Epidermis oder menschlichen Haare gegen UV-Strahlung, speziell im Bereich von 320 bis 400 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hohe spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, die Lichtschutzmittel erfüllen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PARSOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 und EP-A-0 416 837 schon vorgeschlagen, UV-A- und UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

Es bestand daher die Aufgabe, Lichtschutzmittel für kosmetische und pharmazeutische Zwecke vorzuschlagen, die im UV-A-Bereich mit hoher Extinktion absorbieren, die photostabil sind, eine geringe Eigenfarbe d.h. eine scharfe Bandenstrukur aufweisen und je nach Substituent in Öl oder Wasser löslich sind.

Es wurde nun gefunden, daß diese Aufgabe vorteilhaft durch bestimmte Lichtschutzmittelkombinationen gelöst werden kann.

Demgemäß wurde diese Aufgabe erfindungsgemäß gelöst durch die Verwendung von Lichtschutzmittelkombinationen, enthaltend
A im wesentlichen im UV-A-Bereich absorbierende und
B) weitere im UV-A-Bereich, im UV-B-Bereich und in beiden Bereichen absorbierende Verbindungen,
   wobei die im UV-A-Bereich absorbierenden Bestandteile wirksamen Mengen von mindestens
A) einem 4,4'-Diarylbutadien der Formel I 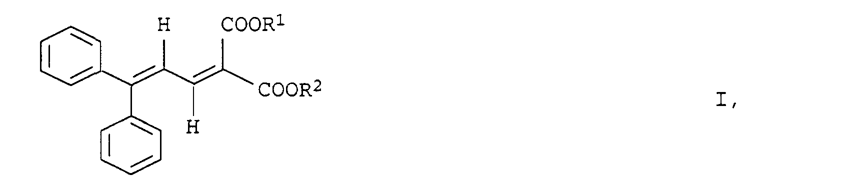 in der
   R¹ und R² unabhängig voneinander Wasserstoff, C₁-C₂₀-Alkyl, C₃-C₁₀-Cycloalkyl oder C₃-C₁₀-Cycloalkenyl bedeuten, enthalten
   und die Bestandteile
B) eine wirksame Menge mindestens einer Verbindung enthalten, ausgewählt aus der Gruppe bestehend aus
Ba) Dibenzoylmethanverbindungen der Formel III 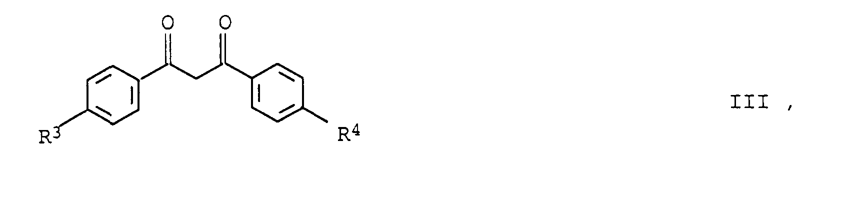 in der
   R³ C₁-C₁₂-Alkyl und
   R⁴ Wasserstoff, C₁-C₁₂-Alkyl oder C₁-C₁₂-Alkoxy bedeuten,
Bb) Triazinderivaten der Formel IV 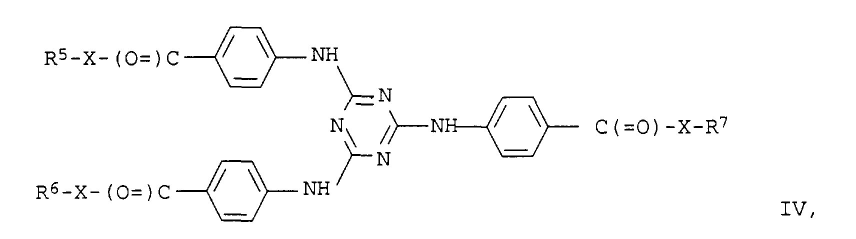 in der
   R⁵ bis R⁷ unabhängig voneinander gegebenenfalls substituiertes C₁-C₂₀-Alkyl, C₅-C₁₀-Aryl, C₅-C₁₀-Heteroaryl oder SpSil bedeuten, wobei Sp für einen Spacer und Sil für einen Silan-, Oligosilan- oder Polysiloxanrest steht
   X für die zweiwertigen Reste
   ―O― oder 〉N-R⁸
   steht, wobei
   R⁸ Wasserstoff oder gegebenenfalls substituiertes C₁-C₂₀-Alkyl, C₅-C₁₀-Aryl oder C₅-C₁₀-Heteroaryl bedeutet,
Bc) dem Triazinderivat der Formel V 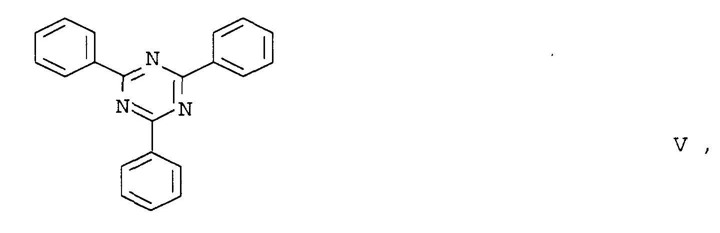 in der an die Phenylringe mindestens eine o-Hydroxygruppe und mindestens eine p-Alkoxygruppe mit 1 bis 20 C-Atomen gebunden sind,
Bd) dem Benztriazolderivat der Formel VI 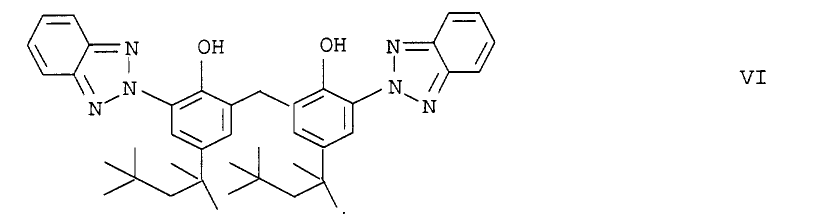
Be) dem Benzimidazolderivat der Formel VII 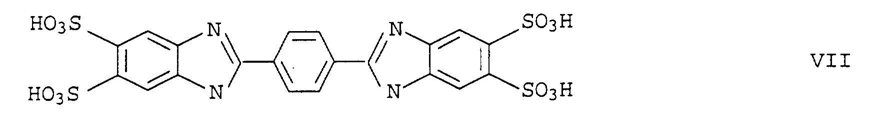 und Salze
Bf) dem Benztriazolderivat der Formel VIII 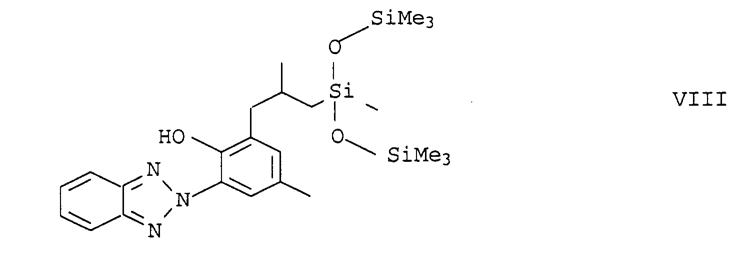
Bg) o,o',p,p'-Tetrahydroxybenzophenon der Formel IX 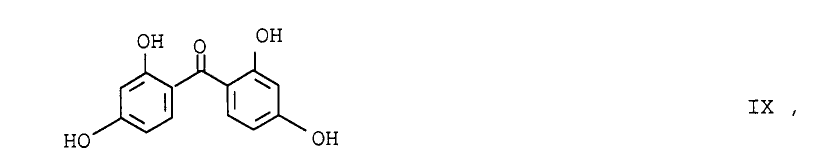
Bh) einem Organosiloxanbenzalmalonat der Formel Xa 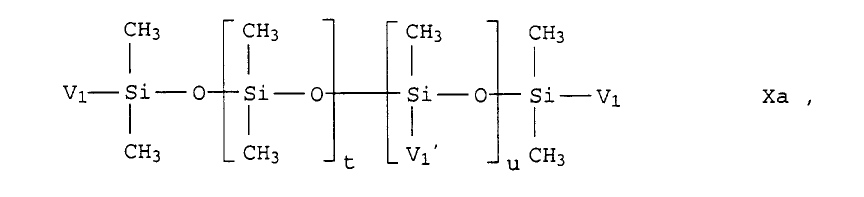 in der
   V₁' die Gruppe 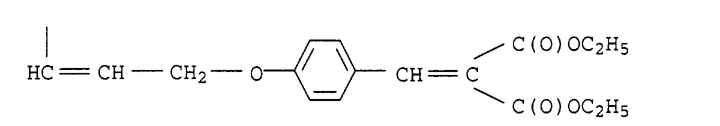 V₁ eine Methylgruppe oder V₁' bedeutet oder
   der Formel Xb 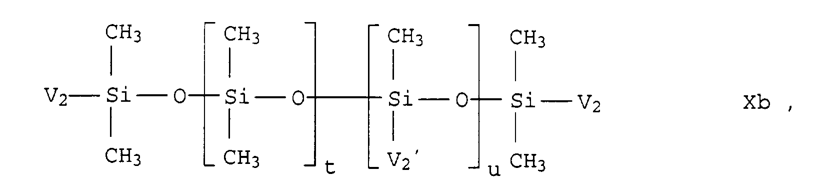 in der V₂' die Gruppe der Struktur 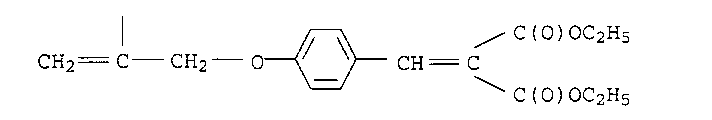 V₂ eine Methylgruppe oder V₂' bedeutet
   oder Mischungen aus Verbindungen der Formeln Xa und Xb,
   wobei t für einen Wert bis 100 und u für einen Wert bis 20 steht mit der Maßgabe, daß u = 0 ist, wenn V₁ = V₁' und/oder V₂ = V'₂ ist und u einen Wert von 1 bis 20 bedeutet, wenn V₁ = CH₃ und/oder V₂ = CH₃ ist,
als photostabile UV-Filter in kosmetischen und pharmazeutischen Zubereitungen zum Schutz der menschlichen Haut der menschlicher Haare gegen Sonnenstrahlen, gegebenenfalls zusammen mit weiteren an sich für kosmetische und pharmazeutische Zubereitungen bekannten, im UV-Bereich absorbierenden Verbindungen.

Die Lichtschutzmittelkombination aus den Verbindungen (A) und (B) kann allein oder in Verbindung mit weiteren im UV-Bereich absorbierenden Verbindungen angewandt werden, doch sollen mindestens wirksame Mengen der Verbindungen (A) bzw. (B) in den Lichtschutzpräparaten enthalten sein. Unter wirksamen Mengen der Verbindungen (A) und der Verbindungen (B) werden im allgemeinen jeweils mindestens 0,2 Gew.-%, jeweils bezogen auf die kosmetische Zubereitung, verstanden.

In den erfindungsgemäßen Lichtschutzmittelkombinationen überwiegt in der Regel die Menge der im UV-B-Bereich absorbierenden Verbindungen. Demgemäß beträgt der Gehalt der im UV-A-Bereich absorbierenden Verbindung (A) im allgemeinen 5 bis 50 Gew.-%, vorzugsweise 10 bis 25 Gew.-%, jeweils bezogen auf Lichtschutzmittelkombination aus (A) und (B).

Die erfindungsgemäßen Kombinationen der Lichtschutzmittel (A) und (B) weisen synergistische Effekte in der Lichtschutzwirkung dergestalt auf, daß die Schutzwirkung der Kombinationen die Summe der Wirkung der Bestandteile übersteigt.

Die Verbindungen (A), das sind 4,4'-Diarylbutadiene der Formel I, 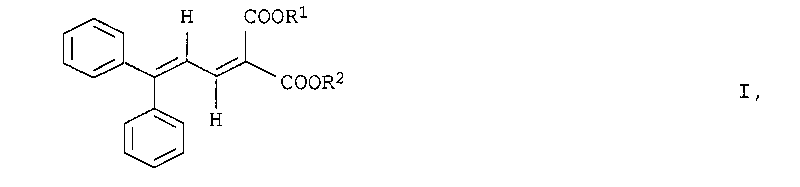 sind aus EPA 916 335 bekannt. Die Substituenten R¹ und/oder R² bedeuten bevorzugt C₁-C₈-Alkyl und C₅-C₈-Cycloalkyl und ganz besonders Neopentyl.

Die Verbindungen (B) sind sämtlich bekannt und werden im folgenden näher charakterisiert:

### (Ba)

Die Verbindungen der Formel III sind aus FR 2440933 bekannt. Als bevorzugte Verbindung der Formel III kommt p-Methoxy-p'-t.-butyldibenzoylmethan (R³ = Methoxy, R⁴ = t.-Butyl) in Betracht.

### (Bb)

Die Verbindungen der Formel IV mit der Bedeutung von R⁵ bis R⁷ Alkyl, Aryl oder Heteroaryl, sind aus EP-A 0796851, EP-A 0087098 und EP-A 0850935 bekannt. Als Alkylreste kommen dabei insbesondere geradkettige oder verzweigte C₁- bis C₁₂-, insbesondere C₁- bis C₈-Reste in Betracht. Arylreste sind z.B. Phenyl- oder Naphthylreste, insbesondere Phenylreste. Als Heteroarylreste kommen einfache oder kondensierte Ringsysteme mit einem oder mehreren heteroaromatischen 3 bis 6-gliedrigen Ringen in Betracht. Als Heteroatome können ein oder mehrere Stickstoff-, Schwefel- und/oder Sauerstoffatome im Ring oder Ringsystem enthalten sein.

Die Verbindungen der Formel IV, mit der Bedeutung SpSil sind aus EPA 0 933 376 bekannt.

Der Begriff Spacer für Sp bedeutet in diesem Zusammenhang eine bivalente verzweigte oder unverzweigte C₃-C₁₂-Alkylen- oder Alkenylenkette, die den Silan-, Oligosiloxan- oder Polysiloxanteil mit dem Triazinrest verknüpft.

Beispiele für eine C₃-C₁₂-Alkylenkette sind Propylen, 2-Methylpropylen, Butylen, Pentylen und Hexylen.

Beispiele für eine C₃-C₁₂-Alkenylenkette sind 2-Propen-2-ylen, 2-Methyl-3-propenylen, 3-Buten-3-ylen und 4-Penten-4-ylen.

Bevorzugte Spacer sind -(CH₂)₃-, -(CH₂)₄-, -(CH₂)₅-, -[CH(CH₃)]-(CH₂)-, -(CH₂)₂-CH=CH-, -C(=CH₂)-CH₂-, -C(=CH₂)-(CH₂)₂-O-(CH₂)₄-, -(CH₂)₄-O-(CH₂)₂-.

Der Begriff Silane steht in diesem Zusammenhang für einen Rest SiR⁹R¹⁰R¹¹, in der R⁹, R¹⁰, R¹¹ unabhängig voneinander für C₁-C₆-Alkyl, C₁-C₆-Alkoxy oder Phenyl stehen.

Als Beispiele seien zu nennen: Si(CH₂-CH₃)₃, Si(CH₂-CH₂-CH₃)₃, Si(isopropyl)₃, Si(ter.butyl)₃, Si(tert.butyl)(CH₃)₂, Si(CH₃)₂ (hexyl), Si(OCH₃)₃, Si(OEt)₃, SiPh₃.

Der Begriff Oligosiloxane bedeutet einen Rest aus der Gruppe der allgemeinen Formel, bestehend aus SiR¹²ₘ(OSiR¹²₃)ₙ mit m=0, 1 oder 2; n=3, 2 oder 1 und m+n=3, R¹²-[Si(R¹²)₂-O-]ᵣ-Si(R¹²)₂-A und R¹²-[Si(R¹²)₂-O-]ᵣ-Si(A)(R¹²)-O-Si(R¹²)₃, in denen A eine chemische Bindung oder einen Spacer und R¹⁵ einen C₁-C₆-Alkylrest oder Phenylrest bedeutet und r für Werte von 1 bis 9 steht.

Der Begriff Polysiloxane beinhaltet beispielsweise einen Rest aus der Gruppe der allgemeinen Formel, bestehend aus
A-[Si(R¹³)₂-O]ₛ-Si(R¹³)₂-A oder
(R¹³)₃-Si-[O-Si(R¹³)₂]]ₜ-[O-Si(R¹³)(A)]_{q}-O-Si(R¹³)₃, in denen A eine chemische Bindung oder einen Spacer und R¹⁶ einen C₁-C₆-Alkylrest oder Phenylrest bedeutet, s und t für Werte von 4 bis 250 und q für Werte von 1 bis 30 stehen.

Als Verbindung der Formel IV kommen vorzugsweise die Triazinverbindungen der Formel IVa 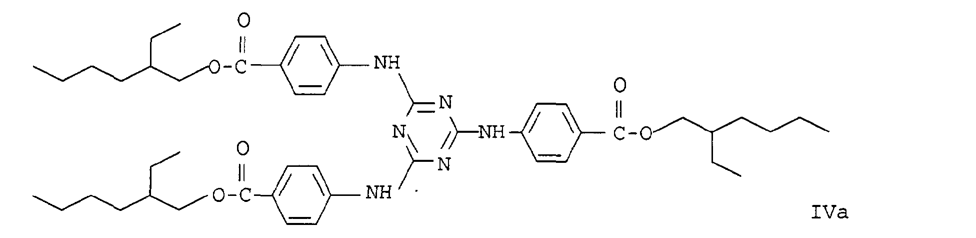 bzw. der Formel IVb 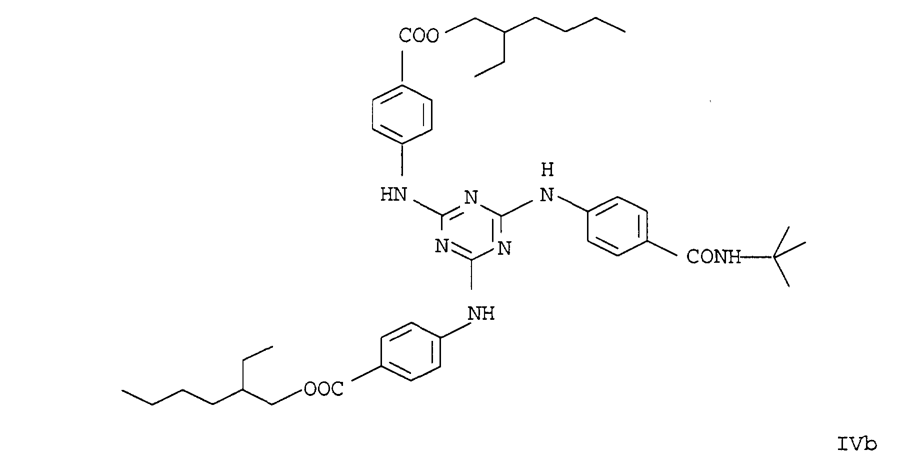 in Betracht.

### (Bc)

Die durch mindestens eine o-Hydroxygruppe und mindestens eine p-Alkoxygruppe substituierten Triphenyltriazine der Formel V sind aus WO 99/66896 bekannt.

Insbesondere kommen Triphenyltriazine der Formel Va in Betracht 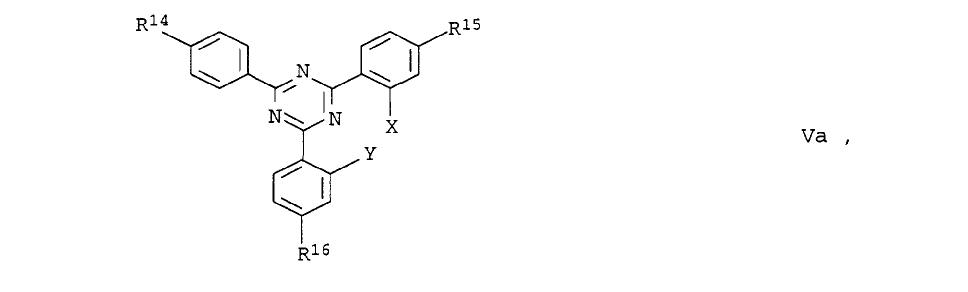 in der X Wasserstoff oder eine Hydroxygruppe und Y eine Hydroxygruppe,
R¹⁴ Wasserstoff oder Alkoxy mit 1 bis 12 C-Atomen und
R¹⁵ und R¹⁶ Alkoxy mit 1 bis 12 C-Atomen bedeuten.

Besonders bevorzugt ist die Verbindung der Formel Vb

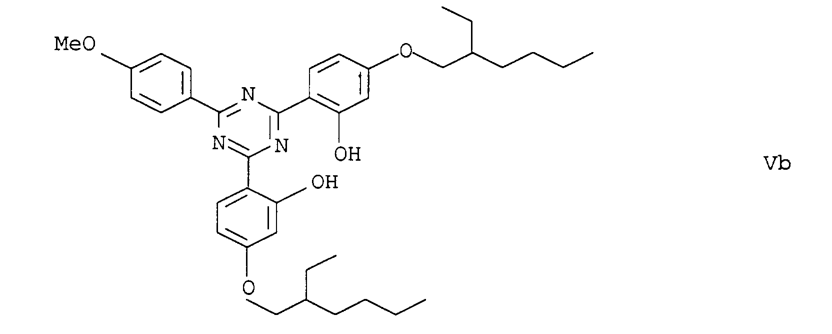

### (Bd)

Die Verbindung der Formel VI hat die CAS Nr. 103597-45-1.

### (Be)

Die Verbindung der Formel VII hat die CAS Nr. 180898-37-7.

### (Bf)

Die Verbindung der Formel VIII hat die CAS Nr. 155633-54-8.

### (Bg)

Die Verbindung der Formel IX hat die CAS Nr. 131-55-5.

### (Bh)

Die Verbindungen der Formeln Xa und Xb sind aus EP-A 0920859 bekannt.

Von den Verbindungen der Formeln Xa und/oder Xb X kommen vor allem die mit den CAS Nummern 208391-15-5, 208391-15-5D, 177955-90-7, 177955-90-7D und 177995-90-7DP in Betracht.

In den erfindungsgemäß zu verwendenden Lichtschutzmittelkombinationen können nicht nur einzelne der Verbindungen (Ba) bis (Bh), sondern auch Mischungen aus mehreren dieser Verbindungen enthalten sein.

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung einer Lichtschutzmittelkombination aufweisen, enthaltend
A) im wesentlichen im UV-A-Bereich absorbierende und
B) weitere im UV-A-Bereich, im UV-B-Bereich und über beide Bereiche absorbierende Verbindungen,
   wobei die im UV-A-Bereich absorbierenden Bestandteile wirksame Mengen von mindestens

A) einem 4,4'-Diarylbutadien der Formel I 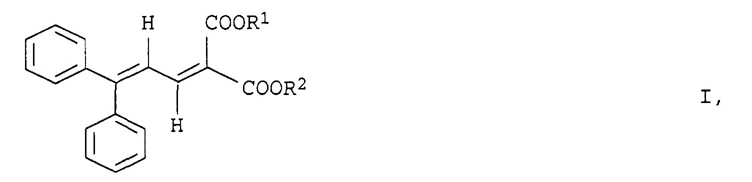 in der
   R¹ und R² unabhängig voneinander Wasserstoff, C₁-C₂₀-Alkyl, C₃-C₁₀-Cycloalkyl oder C₃-C₁₀-Cycloalkenyl bedeuten, enthalten
   und als Bestandteile
B) eine wirksame Menge mindestens einer Verbindung, ausgewählt aus der Gruppe bestehend aus
Ba) Dibenzoylmethanverbindungen der Formel III 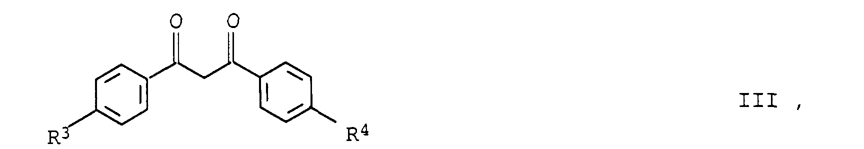 in der
   R³ C₁-C₁₂-Alkyl und
   R⁴ Wasserstoff, C₁-C₁₂-Alkyl oder C₁-C₁₂-Alkoxy bedeuten,
Bb) Triazinderivaten der Formel IV 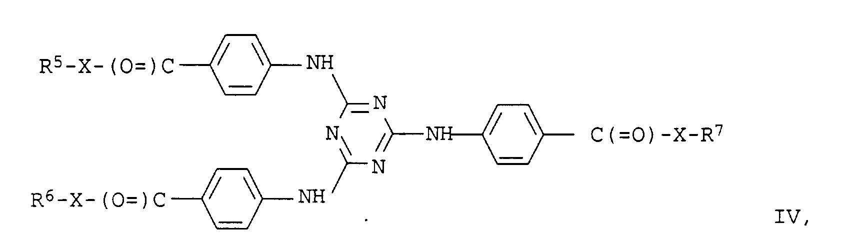 R⁵ bis R⁷ unabhängig voneinander gegebenenfalls substituiertes C₁-C₂₀-Alkyl, C₅-C₁₀-Aryl, C₅-C₁₀-Heteroaryl oder SpSil bedeuten, wobei Sp für einen Spacer und Sil für einen Silan-, Oligosilan- oder Polysiloxanrest steht,
   X für die zweiwertigen Reste
   ―O― oder 〉N-R⁸
   steht, wobei
   R⁸ Wasserstoff oder gegebenenfalls substituiertes C₁-C₂₀-Alkyl, C₅-C₁₀-Aryl oder C₅-C₁₀-Heteroaryl bedeutet,
Bc) Triazinderivaten der Formel V 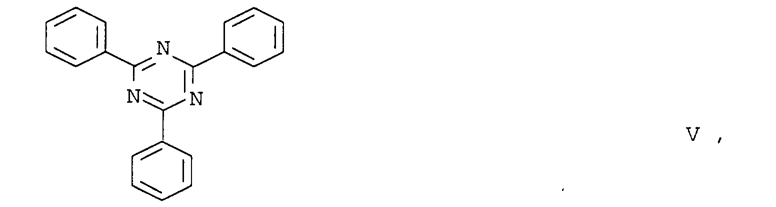 in der an die Phenylringe mindestens eine o-Hydroxygruppe und mindestens eine p-Alkoxygruppe mit 1 bis 20 C-Atomen gebunden sind,
Bd) dem Benztriazolderivat der Formel VI 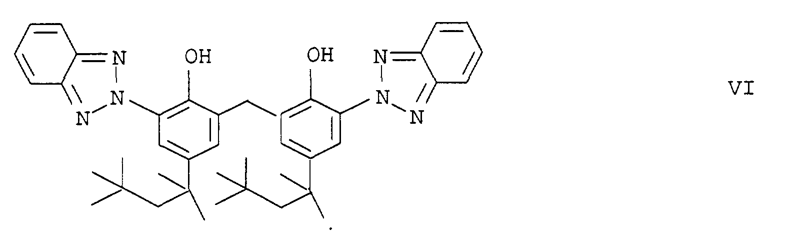
Be) dem Benzimidazolderivat der Formel VII 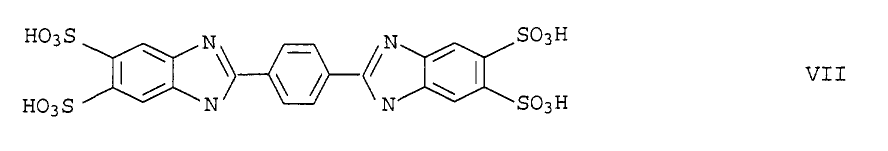 und Salze
Bf) dem Benztriazolderivat der Formel VIII 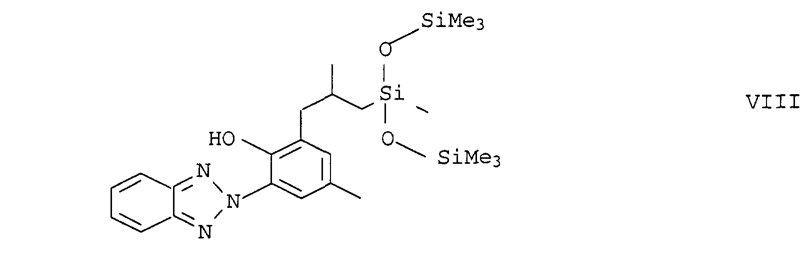
Bg) o,o',p,p'-Tetrahydroxybenzophenon der Formel IX 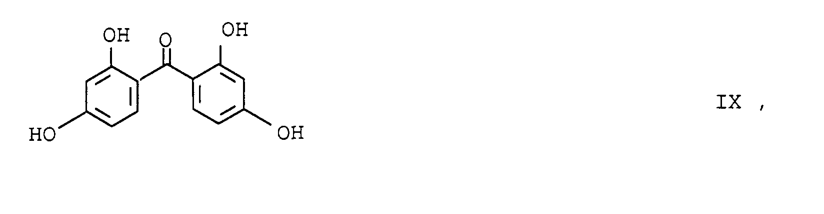
Bh) einem Organosiloxanbenzalmalonat der Formel Xa 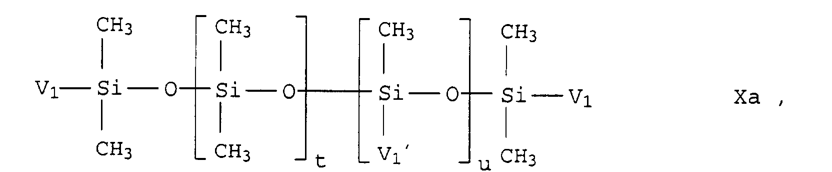 in der
   V₁' die Gruppe 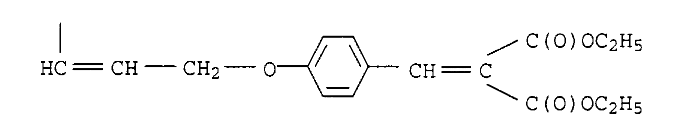 V₁ eine Methylgruppe oder V₁' bedeutet oder
   der Formel Xb 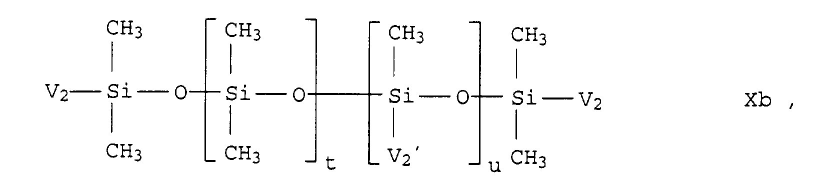 in der V₂' die Gruppe der Struktur 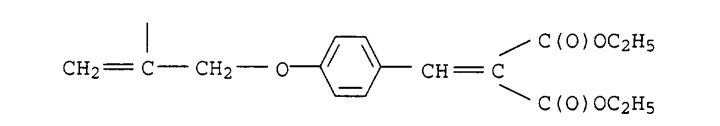 V₂ eine Methylgruppe oder V₂' bedeutet
   oder Mischungen aus Verbindungen der Formeln Xa und Xb,
   wobei t für einen Wert bis 100 und u für einen Wert bis 20 steht mit der Maßgabe, daß u = 0 ist, wenn V₁ = V₁' und/oder V₂ = V'₂ ist und u einen Wert von 1 bis 20 bedeutet, wenn V₁ = CH₃ und/oder V₂ = CH₃ ist,
gegebenenfalls zusammen mit weiteren an sich für kosmetische und pharmazeutische Zubereitungen bekannten, im UV-A- und UV-B-Bereich absorbierenden Verbindungen als Lichtschutzmittel, wobei die UV-A absorbierenden Verbindungen in der Regel in geringerer Menge als die UV-B-absorbierenden Verbindungen eingesetzt werden.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Als Emulsionen kommen u.a. auch O/W-Makroemulsionen, O/W-Mikroemulsionen oder O/W/O-Emulsionen mit in dispergierter Form vorliegenden aminosubstituierten Hydroxybenzophenonen der Formel I in Frage, wobei die Emulsionen durch Phaseninversionstechnologie, gemäß DE-A-197 26 121 erhältlich sind.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium-und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxy-benzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Ein zusätzlicher Gehalt an Antioxidantien ist im allgemeinen bevorzugt. So können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. ß-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Sofern Vitamin A und/oder dessen Derivate bzw. Carotinoide das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Als UV-Filtersubstanzen, die zusätzlich mit den erfindungsgemäß zu verwendenden Lichtschutzmittelkombinationen angewandt werden können, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-onmethylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon(Sulisobenzonum) und das Natriumsalz | 4065-45-6 |
| 13 | 3-(4'-Sulfo)benzyliden-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4'-methoxy-dibenzoylmethan | 70356-09-1 |
| 29 | 2,2',4,4'-Tetrahydroxybenzophenon | 131-55-5 |
| 30 | 2,2'-Methylen-bis-[6(2H-benzotriazol-2-yl)-4-(1,1,3,3,-tetramethylbutyl)phenol] | 103597-45-1 |
| 31 | 2,2'-(1,4-Phenylen)-bis-1H-benzimidazol-4,6-disulfonsäure, Na-Salz | 180898-37-7 |
| 32 | 2,4-bis-[4-(2-Ethylhexyloxy)-2-hydroxy]phenyl-6-(4-methoxyphenyl)-(1,3,5)-triazin | 187393-00-6 |

Die erfindungsgemäßen kosmetischen und dermatologischen Zubereitungen können vorteilhafterweise außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten. Besonders bevorzugt handelt es sich um Pigmente auf der Basis TiO₂ und ZnO.

Es ist besonders vorteilhaft im Sinne der vorliegenden Erfindung, wenngleich nicht zwingend, wenn die anorganischen Pigmente in hydrophober Form vorliegen, d.h. daß sie oberflächlich wasserabweisend behandelt sind. Diese Oberflächenbehandlung kann darin bestehen, daß die Pigmente nach an sich bekannter Weise, wie in DE-A-33 14 742 beschrieben, mit einer dünnen hydrophoben Schicht versehen sind.

Zum Schutz menschlicher Haare vor UV-Strahlen können die erfindungsgemäß zu verwendenden Lichtschutzmittelkombinationen in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Lichtschutzmittelkombinationen sind gut in kosmetischen Ölen löslich und lassen sich leicht in kosmetische Formulierungen einarbeiten. Die mit den neuen Verbindungen Lichtschutzmittelkombinationen hergestellten Emulsionen zeichnen sich besonders durch ihre hohe Stabilität, die Lichtschutzmittelkombinationen selber durch ihre hohe Photostabilität aus, und die mit den Lichtschutzmittelkombinationen hergestellten Zubereitungen durch ihr angenehmes Hautgefühl aus.

Die UV-Filterwirkung der erfindungsgemäß zu verwendenden Lichtschutzmittelkombinationen kann auch zur Stabilisierung von Wirk- und Hilfsstoffen in kosmetischen und pharmazeutischen Formulierungen ausgenutzt werden.

In den folgenden Beispielen wird die Verwendung der neuen Lichtschutzmittelkombinationen näher erläutert.

### Beispiele

### Beispiel 1 - Standardisierte Methode zur Bestimmung der Photostabilität (Suntest)

Eine 5 Gew.-%ige alkoholische Lösung des zu prüfenden Lichtschutzmittels wird mittels einer Eppendorfpipette (20 µl) auf die Auffräsung eines Glasplättchens aufgetragen. Durch die Anwesenheit des Alkohols verteilt sich die Lösung gleichmäßig auf der aufgerauten Glasoberfläche. Die aufgetragene Menge entspricht der Menge an Lichtschutzmittel, die in Sonnencremes zur Erreichung eines mittleren Lichtschutzfaktors benötigt wird. Bei der Prüfung werden jeweils 4 Glasplättchen bestrahlt. Die Abdampfzeit und die Bestrahlung betragen je 30 Minuten. Die Glasplättchen werden während des Bestrahlens durch eine Wasserkühlung, die sich am Boden des Suntestgeräte befindet, leicht gekühlt. Die Temperatur innerhalb des Suntest Gerätes beträgt während der Bestrahlung 40°C. Nachdem die Proben bestrahlt worden sind, werden sie mit Ethanol in einen dunklen 50 ml Meßkolben gewaschen und mit dem Photometer vermessen. Die Blindproben werden ebenso auf Glasplättchen aufgetragen und 30 Minuten bei Raumtemperatur abgedampft. Wie die anderen Proben werden sie mit Ethanol abgewaschen und auf 100 ml verdünnt und vermessen.

Allgemeine Vorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

Zubereitungen

### Beispiel 2 - Zusammensetzung für die Lippenpfleqe

| Massengehalt (Gew.-%) | |
|---|---|
| ad 100 | Eucerinum anhydricum |
| 10,00 | Glycerin |
| 10,00 | Titanium Dioxid, mikronisiert |
| 5,00 | Verbindungskombination aus 1 % A (Formel I, R = Neopentyl), 1 % Bb (Formel IVa) und 3 % Bf (Formel VIII) |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 3 - Zusammensetzung für die Lippenpflege

| Massengehalt (Gew.-%) | |
|---|---|
| ad 100 | Eucerinum anhydricum |
| 10,00 | Glycerin |
| 10,00 | Titanium Dioxid, mikronisiert |
| 5,00 | Verbindungskombination aus 1 % A (Formel I, R = Neopentyl) und 4 % Bd (Formel VI) |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 4 - Zusammensetzung für Sunblocker mit Mikropigmenten

| Massengehalt (Gew.-%) | |
|---|---|
| ad 100 | Wasser |
| 10,00 | Octyl Methoxcinnamat |
| 6,00 | PEG-7-Hydrogenated Castor Öl |
| 6,00 | Titanium Dioxid, mikronisiert |
| 5,00 | Verbindungskombination aus 1,5 % A (Formel I, R = Neopentyl), 1,5 % Bb (Formel IVa) und 2 % Bd (Formel VI) |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 5 - Zusammensetzung für Sunblocker mit Mikropigmenten

| Massengehalt (Gew.-%) | |
|---|---|
| ad 100 | Wasser |
| 10,00 | Octyl Methoxcinnamat |
| 6,00 | PEG-7-Hydrogenated Castor Öl |
| 6,00 | Titanium Dioxid, mikronisiert |
| 5,00 | Verbindungskombination bestehend aus 1,5 % A (Formel I, R = Neopentyl) und 3,5 % Bb (Formel IVb) |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 6 - Fettfreies Gel

| Massengehalt (Gew.-%) | |
|---|---|
| ad 100 | Wasser |
| 8,00 | Octyl Methoxycinnamat |
| 7,00 | Titanium Dioxid, mikronisiert |
| 5,00 | Verbindungskombination, bestehend aus 1 % A (Formel I, R = Neopentyl), 2 % Bf (Formel VIII) und 2 % Bc (Formel Vb) |
| 5,00 | Glycerin |
| 5,00 | PEG-25 PABA |
| 1,00 | 4-Methylbenzyliden Campher |
| 0,40 | Acrylate C₁₀-C₃₀ Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 7 - Fettfreies Gel

| Massengehalt (Gew.-%) | |
|---|---|
| ad 100 | Wasser |
| 8,00 | Octyl Methoxycinnamat |
| 7,00 | Titanium Dioxid, mikronisiert |
| 5,00 | Verbindungskombination, bestehend aus 1,5 % A (Formel I, R = Neopentyl), 1 % Bf (Formel VIII) und 2,5 % Bc (Formel Vb) |
| 5,00 | Glycerin |
| 5,00 | PEG-25 PABA |
| 1,00 | 4-Methylbenzyliden Campher |
| 0,40 | Acrylate C₁₀-C₃₀ Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 8 - Sonnencreme

| Massengehalt (Gew.-%) | |
|---|---|
| ad 100 | Wasser |
| 8,00 | Octyl Methoxycinnamat |
| 8,00 | Titanium Dioxid, mikronisiert |
| 6,00 | PEG-7-Hydrogenated Castor Öl |
| 5,00 | Verbindungskombination, bestehend aus 1,5 % A (Formel I, R = Neopentyl), 1,5 % Bg (Formel IX) und 2 % Bb (Formel IVb) |
| 6,00 | Mineral Öl |
| 5,00 | Zink Oxid |
| 5,00 | Isopropyl Palmitat |
| 0,30 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 9 - Sonnencreme

| Massengehalt (Gew.-%) | |
|---|---|
| ad 100 | Wasser |
| 8,00 | Octyl Methoxycinnamat |
| 8,00 | Titanium Dioxid, mikronisiert |
| 6,00 | PEG-7-Hydrogenated Castor Öl |
| 5,00 | Verbindungskombination, bestehend aus 2 % A (Formel I, R = Neopentyl), 1 % Be (Formel VII) und 2 % Bc (Formel Vb) |
| 6,00 | Mineral Öl |
| 5,00 | Zink Oxid |
| 5,00 | Isopropyl Palmitat |
| 0,30 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 10 - Sonnencreme wasserfest

| Massengehalt (Gew.-%) | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindungskombination, bestehend aus 1 % A (Formel I, R = Neopentyl), 2 % Bb (Formel IVa) und 2 % Bd (Formel VI) |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 11 - Sonnencreme wasserfest

| Massengehalt (Gew.-%) | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindungskombination, bestehend aus 1 % A (Formel I, R = Neopentyl), 1 % Bb (Formel IVa), 1 % Ba (Formel III, R₃ = tert.-Butyl, R₄ = Methoxy und 2 % Bc (Formel Vb) |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 12 - Sonnenmilch

| Massengehalt (Gew.-%) | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindungskombination, bestehend aus 1 % A (Formel I, R = Neopentyl) und 4 % Bb (Formel IVb) |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 3,00 | Glycerin |
| 0,25 | Methylparaben |
| 0,15 | Propylparaben |
| 0,05 | Tocopherol |

### Beispiel 13 - Sonnenmilch

| Massengehalt (Gew.-%) | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindungskombination, bestehend aus 0,5 % A (Formel I, R = Neopentyl), 1 % Bb (Formel IVa) und 3,5 % Bc (Formel Vb) |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 3,00 | Glycerin |
| 0,25 | Methylparaben |
| 0,15 | Propylparaben |
| 0,05 | Tocopherol |

## Patentansprüche

1. Verwendung von Lichtschutzmittelkombinationen, enthaltend
A) im wesentlichen im UV-A-Bereich absorbierende und
B) weitere im UV-A-Bereich, im UV-B-Bereich und über beide Bereiche absorbierende Verbindungen,
wobei die im UV-A-Bereich absorbierenden Bestandteile wirksamen Mengen von mindestens
A) einem 4,4'-Diarylbutadien der Formel I 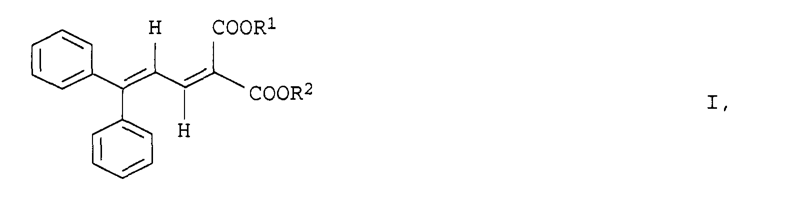 in der
R¹ und R² unabhängig voneinander Wasserstoff,
C₁-C₂₀-Alkyl, C₃-C₁₀-Cycloalkyl oder C₃-C₁₀-Cycloalkenyl bedeuten, enthalten
und als Verbindungen
B) wirksame Mengen mindestens einer Verbindung enthalten, ausgewählt aus der Gruppe bestehend aus
Ba) Dibenzoylmethanverbindungen der Formel III 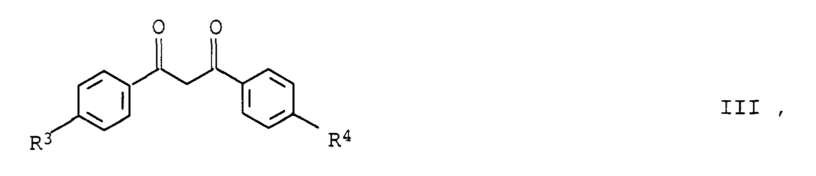 in der
R³ C₁-C₁₂-Alkyl und
R⁴ Wasserstoff, C₁-C₁₂-Alkyl oder C₁-C₁₂-Alkoxy bedeuten,
Bb) Triazinderivaten der Formel IV 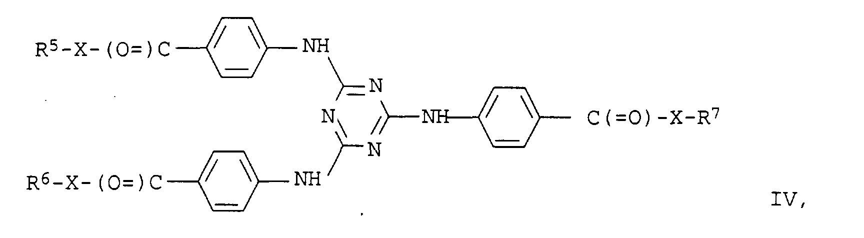 in der
R⁵ bis R⁷ unabhängig voneinander gegebenenfalls substituiertes C₁-C₂₀-Alkyl, C₅-C₁₀-Aryl, C₅-C₁₀-Heteroaryl oder SpSil bedeuten, wobei Sp für einen Spacer und Sil für einen Silan-, Oligosiloxan- oder Polysiloxanrest steht,
X für die zweiwertigen Reste
-O- oder 〉N-R⁸
steht, wobei
R⁸ Wasserstoff oder gegebenenfalls substituiertes C₁-C₂₀-Alkyl, C₅-C₁₀-Aryl oder C₅-C₁₀-Heteroaryl bedeutet,
Bc) Triazinderivaten der Formel V 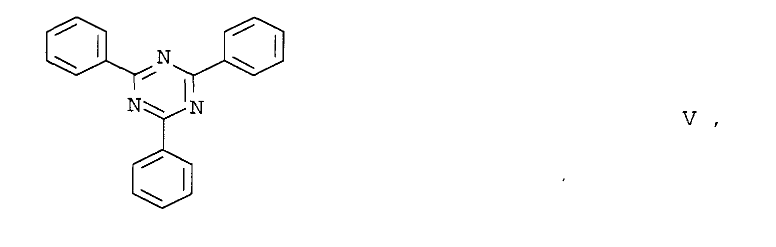 in der an die Phenylringe mindestens eine o-Hydroxygruppe und mindestens eine p-Alkoxygruppe mit 1 bis 20 C-Atomen gebunden sind,
Bd) dem Benztriazolderivat der Formel VI 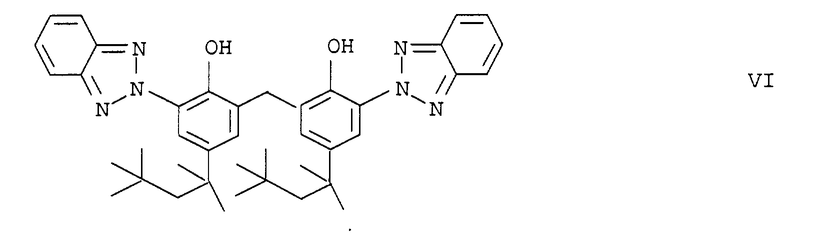
Be) dem Benzimidazolderivat der Formel VII 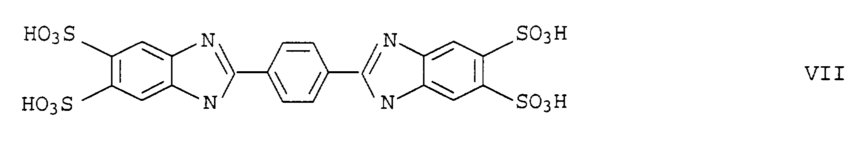 und Salze
Bf) dem Benztriazolderivat der Formel VIII 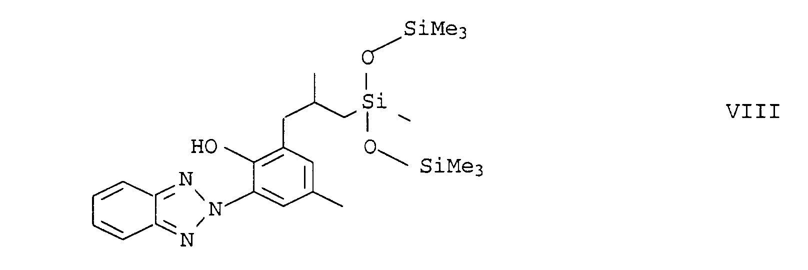
Bg) o,o',p,p'-Tetrahydroxybenzophenon der Formel IX 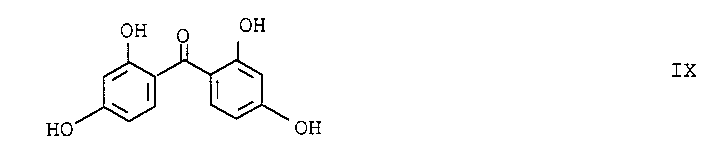 und
Bh) einem Organosiloxanbenzalmalonat der Formel Xa 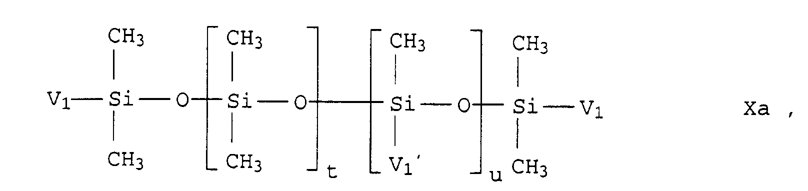 in der
V₁' die Gruppe 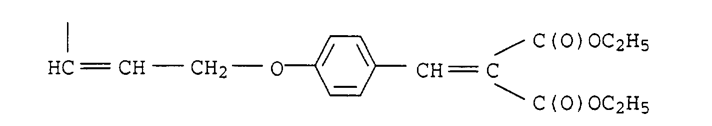 V₁ eine Methylgruppe oder V₁' bedeutet oder
der Formel Xb 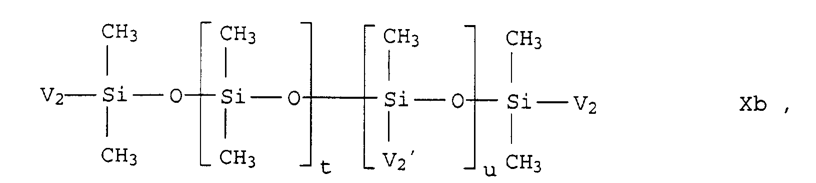 in der V₂' die Gruppe der Struktur 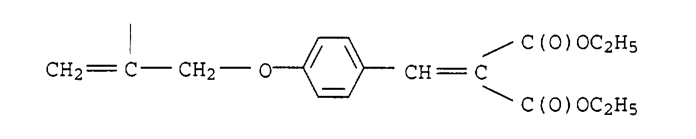 V₂ eine Methylgruppe oder V₂' bedeutet
oder Mischungen aus Verbindungen der Formeln Xa und Xb,
wobei t für einen Wert bis 100 und u für einen Wert bis 20 steht mit der Maßgabe, daß u = 0 ist, wenn V₁ = V₁' und/oder V₂ = V'₂ ist und u einen Wert von 1 bis 20 bedeutet, wenn V₁ = CH₃ und/oder V₂ = CH₃ ist,
als photostabile UV-Filter in kosmetischen und pharmazeutischen Zubereitungen zum Schutz der menschlichen Haut der menschlicher Haare gegen Sonnenstrahlen, gegebenenfalls zusammen mit weiteren an sich für kosmetische und pharmazeutische Zubereitungen bekannten, im UV-Bereich absorbierenden Verbindungen.

2. Verwendung von Lichtschutzmittelkombinationen gemäß Anspruch 1, **dadurch gekennzeichnet, daß** Sie Verbindungen der Formel I enthalten, in der R¹ und/oder R² Neopentyl bedeutet.

3. Verwendung von Lichtschutzmittelkombinationen gemäß Anspruch 1, **dadurch gekennzeichnet, daß** sie als Bestandteil Bb) Triazinderivate der Formel IV enthalten, in der die Reste -X-R⁵ bis -X-R⁷ 2-Ethylhexyloxy bedeuten.

4. Verwendung von Lichtschutzmittelkombinationen gemäß Anspruch 1, **dadurch gekennzeichnet, daß** sie als Bestandteil Bb) Triazinderivate der Formel IV enthalten, in der der Rest -X-R⁵ t-Butylamino und -X-R⁶ und -X-R⁷ den Rest 2-Ethylhexyloxy bedeuten.

5. Verwendung von Lichtschutzmittelkombinationen gemäß Anspruch 1, **dadurch gekennzeichnet, daß** sie den Bestandteil A) der Formel I in Mengen von mindestens 5 Gew.-%, bezogen auf die Lichtschutzmittelkombination, enthalten.

6. Verwendung von Lichtschutzmittelkombinationen gemäß Anspruch 1, **dadurch gekennzeichnet, daß** sie zusätzlich zu B) Pigmente in Form von Zinkoxid oder Titandioxid enthalten.

7. Lichtschutzmittelkombinationen enthaltende kosmetische und pharmazeutische Zubereitungen zum Schutz der menschlichen Epidermis oder der menschlichen Haare gegen UV-Licht im Bereich von 280 bis 400 nm, **dadurch gekennzeichnet, daß** sie in einem kosmetisch und pharmazeutisch geeigneten Träger als photostabile UV-Filter wirksame Mengen von Lichtschutzmittelkombinationen enthalten, die
A) im wesentlichen im UV-A-Bereich absorbierende und
B) weitere im UV-A-Bereich, im UV-B-Bereich und über beide Bereiche absorbierende Verbindungen aufweisen,
wobei die im UV-A-Bereich absorbierenden Bestandteile wirksame Mengen von mindestens
A) einem 4,4'-Diarylbutadien der Formel I 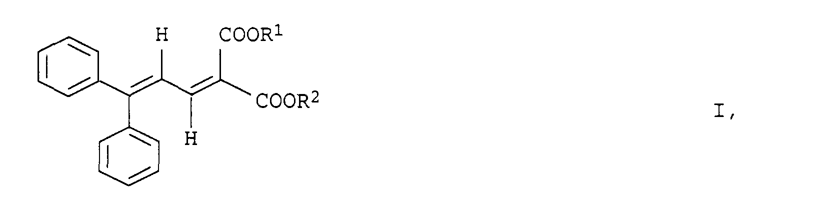 in der
R¹ und R² unabhängig voneinander Wasserstoff,
C₁-C₂₀-Alkyl, C₃-C₁₀-Cycloalkyl oder C₃-C₁₀-Cycloalkenyl bedeuten, enthalten
und die Bestandteile
B) eine wirksame Menge mindestens einer Verbindung aufweisen, ausgewählt aus der Gruppe bestehend aus
Ba) Dibenzoylmethanverbindungen der Formel III 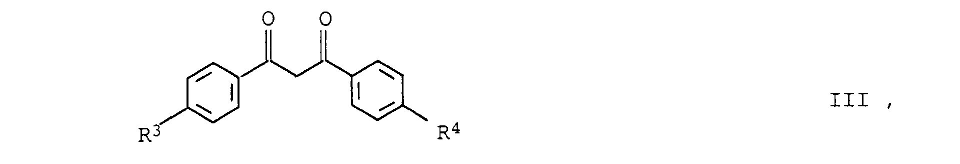 in der
R³ C₁-C₁₂-Alkyl und
R⁴ Wasserstoff, C₁-C₁₂-Alkyl oder C₁-C₁₂-Alkoxy bedeuten,
Bb) Triazinderivaten der Formel IV 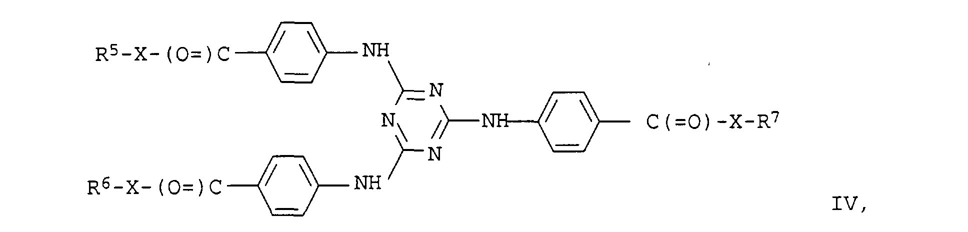 R⁵ bis R⁷ unabhängig voneinander gegebenenfalls substituiertes C₁-C₂₀-Alkyl, C₅-C₁₀-Aryl, C₅-C₁₀-Heteroaryl oder SpSil bedeuten, wobei Sp für einen Spacer und Sil für einen Silan-. Oligosilan- oder Polysiloxanrest steht,
X für die zweiwertigen Reste
-O- oder 〉N-R⁸
steht, wobei
R⁸ Wasserstoff oder gegebenenfalls substituiertes C₁-C₂₀-Alkyl, C₅-C₁₀-Aryl oder C₅-C₁₀-Heteroaryl bedeutet,
Bc) Triazinderivaten der Formel V 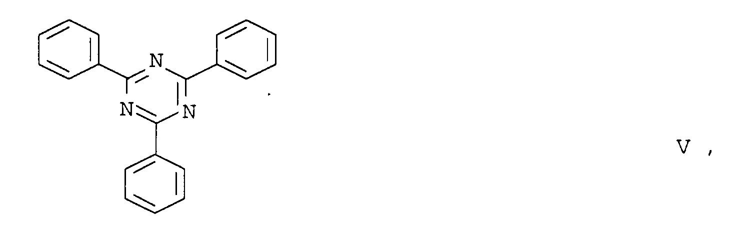 in der an die Phenylringe mindestens eine o-Hydroxygruppe und mindestens eine p-Alkoxygruppe mit 1 bis 20 C-Atomen gebunden sind,
Bd) dem Benztriazolderivat der Formel VI 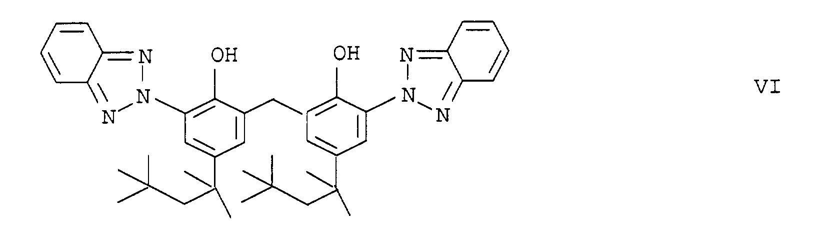
Be) dem Benzimidazolderivat der Formel VII 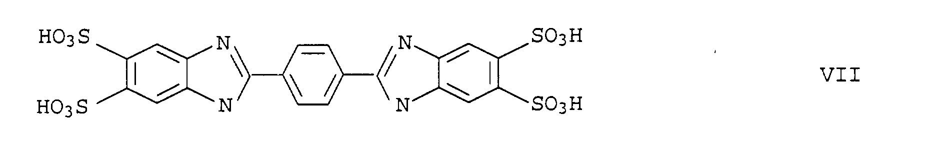 und Salze
Bf) dem Benztriazolderivat der Formel VIII 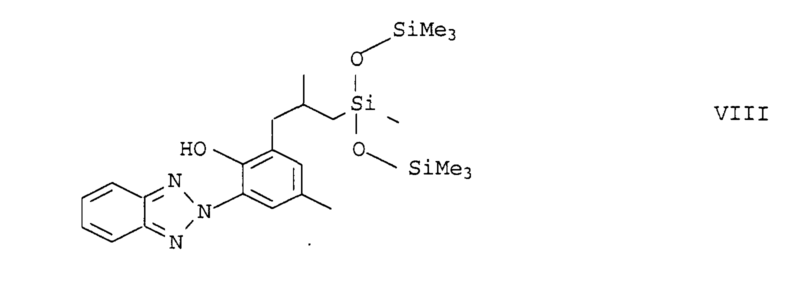
Bg) o,o',p,p'-Tetrahydroxybenzophenon der Formel IX 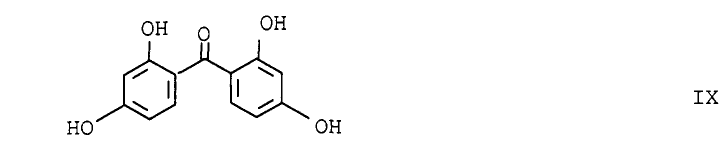 und
Bh) einem Organosiloxanbenzalmalonat der Formel Xa 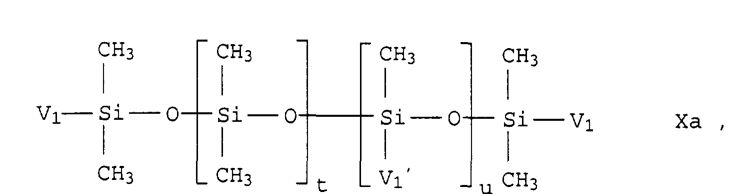 in der
V₁' die Gruppe 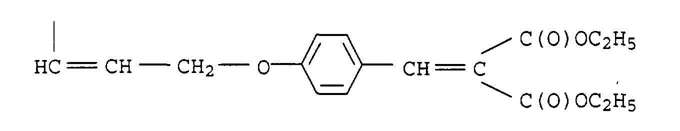 V₁ eine Methylgruppe oder V₁' bedeutet oder
der Formel Xb 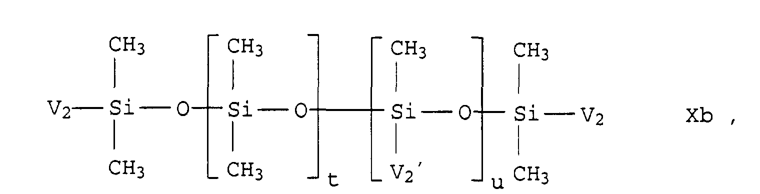 in der V₂' die Gruppe der Struktur 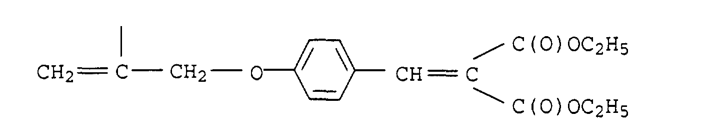 V₂ eine Methylgruppe oder V₂' bedeutet
oder Mischungen aus Verbindungen der Formeln Xa und Xb,
wobei t für einen Wert bis 100 und u für einen Wert bis 20 steht mit der Maßgabe, daß u = 0 ist, wenn V₁ = V₁' und/oder V₂ = V'₂ ist und u einen Wert von 1 bis 20 bedeutet, wenn V₁ = CH₃ und/oder V₂ = CH₃ ist, als photostabile UV-Filter in kosmetischen und pharmazeutischen Zubereitungen zum Schutz der menschlichen Haut der menschlicher Haare gegen Sonnenstrahlen, gegebenenfalls zusammen mit weiteren an sich für kosmetische und pharmazeutische Zubereitungen bekannten, im UV-Bereich absorbierenden Verbindungen,
aufweisen, gegebenenfalls zusammen mit weiteren an sich für kosmetische und pharmazeutische Zubereitungen bekannten, im UV-Bereich absorbierenden Verbindungen.

8. Lichtschutzmittelkombinationen enthaltende kosmetische und pharmazeutische Zubereitungen gemäß Anspruch 7, **dadurch gekennzeichnet, daß** die Lichtschutzmittelkombinationen Verbindungen der Formel I enthalten, in der R¹ und/oder R² Neopentyl bedeutet.

9. Lichtschutzmittelkombinationen enthaltende kosmetische und pharmazeutische Zubereitungen gemäß Anspruch 7, **dadurch gekennzeichnet, daß** die Lichtschutzmittelkombinationen als Bestandteil Bb) Triazinderivate der Formel IV enthalten, in der die Reste -X-R⁵ bis -X-R⁷ 2-Ethylhexyloxy bedeuten.

10. Lichtschutzmittelkombinationen enthaltende kosmetische und pharmazeutische Zubereitungen gemäß Anspruch 7, **dadurch gekennzeichnet, daß** die Lichtschutzmittelkombinationen als Bestandteil Bb) Triazinderivate der Formel IV enthalten, in der der Rest -X-R⁸ t-Butylamino und -X-R⁹ und -X-R¹⁰ den Rest 2-Ethylhexyloxy bedeuten.

11. Lichtschutzmittelkombinationen enthaltende kosmetische und pharmazeutische Zubereitungen gemäß Anspruch 7, **dadurch gekennzeichnet, daß** die Lichtschutzmittelkombinationen als Bestandteil der Formel I in Mengen von mindestens 5 Gew.-%, bezogen auf die Lichtschutzmittelkombination, enthalten.

12. Lichtschutzmittelkombinationen enthaltende kosmetische und pharmazeutische Zubereitungen gemäß Anspruch 7, **dadurch gekennzeichnet, daß** die Lichtschutzmittelkombinationen zusätzlich zu B) Pigmente in Form von Zinkoxid oder Titandioxid enthalten.
